# EUROPEAN PATENT SPECIFICATION

(11) **EP 3 687 656 B1**
(45) Date of publication and mention of the grant of the patent: **30.03.2022**
(21) Application number: 18849465.2
(22) Date of filing: 20.12.2018
(51) Int. Cl.: B01D 39/16, C12N 5/074

(54) **MEMBRANE FOR SEPARATION OF STEM CELLS FROM BIOLOGICAL SAMPLES, PRODUCTION PROCESS OF SAID MEMBRANE, AND PROCESS AND DEVICE FOR SEPARATION, COMPRISING SAID MEMBRANE**
MEMBRAN ZUR TRENNUNG VON STAMMZELLEN AUS BIOLOGISCHEN PROBEN, HERSTELLUNGSVERFAHREN DAFÜR SOWIE VERFAHREN UND VORRICHTUNG ZUR TRENNUNG MIT DIESER MEMBRAN
MEMBRANE POUR LA SÉPARATION DE CELLULES SOUCHES D'ÉCHANTILLONS BIOLOGIQUES, PROCESSUS DE PRODUCTION DE LADITE MEMBRANE, ET PROCESSUS ET DISPOSITIF DE SÉPARATION, COMPRENANT LADITE MEMBRANE

(43) Date of publication of application: 05.08.2020
(73) Proprietor: Bio-ReCell Ltd., London, EC3M 6BL (GB)
(72) Inventor: ARKO, Zoran, 1261 Ljubljana Dobrunje (SI); TOFANT, Tadej, 2250 Ptuj (SI); SIMONCIC, Boris, 1000 Ljubljana (SI); MAVER, Uros, 2313 Fram (SI); MAVER, Tina, 2313 Fram (SI); GOLE, Boris, 1241 Kamnik (SI); POTOCNIK, Uros, 2345 Bistrica ob Dravi (SI); ZIDARIC, Tanja, 2000 Maribor (SI)
(74) Representative: Jersan, Tatjana
(86) International application number: PCT/SI2018/050035
(87) International publication number: WO 2020/130949

(56) References cited:
- EP-A1- 1 947 170
- CN-U- 205 687 938
- NICODEMOU ANDREAS ET AL: "Mesenchymal stromal/stem cell separation methods: concise review", CELL AND TISSUE BANKING, SPRINGER, NL, vol. 18, no. 4, 18 August 2017 (2017-08-18), pages 443-460, XP036356818, ISSN: 1389-9333, DOI: 10.1007/S10561-017-9658-X [retrieved on 2017-08-18]

## Description

The present invention is a membrane for the separation of target stem cells from biological samples - more precisely from a single-cell suspension prepared from a biological sample - thus obtaining sterile target stem cells in a physiological buffer of a known cell population size (number of isolated cells) and viability (live/dead cells ratio). Thus obtained target stem cells may be used either in therapy - immediately after separation or subsequently for the development of tissue fillers or new solutions in regenerative medicine, related to various tissues in dental medicine, orthopedics, plastic surgery, etc. or in research, for example the study of stem cell biology or testing of new therapeutic agents, etc. The membrane is designed as a 3D carrier structure made of at least one layer of a biocompatible polymer with predefined pore size as a carrier material, featuring covalently bound target molecules, preferably target antibodies - either on its surface and/or in the pores - recognizing characteristic antigens bound to the surface of target stem cells and thus binding the target stem cells to the membrane. Target molecules can be bound directly to the surface and/or in the pores of the carrier structure or they can be bound to or integrated in the 3D membrane structure by specific functionalized nanoparticles.

In addition, the present invention includes the membrane production process as well as the process and device for the separation of target stem cells from a biological sample, which includes the above membrane as a constituent part.

The use of the membrane and the processes of the invention enable a highly specific and effective active separation of target stem cells from the cell mixture in a biological sample.

State of the art, technical problems, and deficiencies solved by this invention.

US patent no. 794266 refers to the isolation of cells by binding magnetic particles to the cells. The solutions from the above patent are not comparable with the solutions disclosed in the present invention, for the above patent uses a cell separation technology based on the magnetic attractive force in a field, the present invention however induces cell separation exclusively based on the reaction of the antibody with the cell.

US patent no. 7592431 refers to the isolation of T_{reg} cells using biocompatible carrier structures and activation of cell surface markers. Relevant literature does not imply any sufficiently specific markers in T_{reg} cells; therefore, the process according to the invention is based on a different approach: differentiation of stem cells into Treg cells.

Patent application no.WO2017075389 includes the description of obtaining cells using corresponding markers; the authors did not specify the type of input-tissue; the procedure is carried out manually (takes more time, money and the yields are not comparable) and antibodies used are not comparable to the antibodies mentioned in the present invention (e.g. CD90...).

US patent no. 7390484 includes the description of a new optimized collection container for lipoaspiration, including a filter system enabling the enrichment of cell suspension. It is about cell "concentration" and the application thereof onto cell culture plates for further purification and multiplication of cells obtained from the sample.

US patent application no. 20130130371 describes mechanic purification of lipoaspirate using mesh filters. However, the procedure according to the invention determines cell suspension as input material, which can be prepared in several ways, therefore the procedure according to the present invention is not limited to filtering or purification using mesh filters. Moreover, the above patent application does not mention any "affinity-based cell-separation" procedure. It mentions the prepreparation of the lipoaspirate for further use.

US patent application no. 20130034524 describes the generally known protocol for cell isolation using centrifugation, enzymatic digestion, etc. It does not interfere with the invention presented, for the above patent application refers to "cell enrichment or concentration"- not to cell isolation or separation. This means as well that the above patent applied for is not about an affinity-based separation method; also, the yields are substantially lower than the yields of the invention presented, especially because even after centrifugation they still obtain a "mixture of cells".

EP patent application No. 1947170 discloses a stem cell separating material as well as a method of separation, wherein essentially a basic filtering method is disclosed, where all stem cells, which are present in a biological sample, adhere to the surface of the material.

CN utility model No. 205687938U discloses a device for the separation of adipose stem cells from a mixed cell suspension.

Different methods for separation of mesenchymal stem cells are disclosed in Article "Mesenchymal stromal/stem cell separation methods: concise review" from Nicodemou Andreas et al.

The above mentioned deficiencies of the state-of-the-art technologies are solved by a membrane and a separation process according to the invention.

Within the context of this application, the term "stem cells" defines cells with a great (theoretically infinite) ability of population self-renewal (meaning they can divide in such a way that more cells of the same type are formed), which can be differentiated into at least one other cell type, thus having the ability to repopulate or regenerate (various) tissues after transplantation. At the molecular level, these cells express the so-called stem cell markers, such as surface antigens characteristic for stem cells. Depending on the biological sample, stem cells contained therein express characteristic surface antigens. For example, hematopoietic stem cells isolated from peripheral or umbilical-cord blood express among other CD34 surface antigens, whereas mesenchymal stem cells isolated from adipose tissue express CD90 surface antigens.

The term "target stem cell" means stem cells expressing characteristic surface antigens that bind to selected target molecules.

The term "target molecule" refers to a molecule that recognizes the characteristic antigen on the surface of the target stem cell, and can bind onto this characteristic antigen. In the process, the target molecule should not affect the target stem cell itself, that is for example its differentiation, or forcing it to divide, and, when used, should not affect the secretion of any substances that could induce momentary (acute) or long-term (chronic) negative impacts on the patient or influence the characteristics (genotype, epigenetic or phenotype) of target stem cells. Preferentially, a target molecule is the entire antibody or part of the antibody that recognizes the characteristic antigen on the surface of the target stem cell (e.g. region Fc, region Fab, aptamer) and enables specific binding to the characteristic antigen. Preferentially, these are antibodies that recognize the following antigens: CD90, CD146, CD44, CD73, CD105, CD34, STRO-1, STRO-3, etc.

The biological sample for the separation of target stem cells can be any human and animal organs, tissues, and fluids that include such cells and are taken from living or dead donors. These are above all, but not limited to: subcutaneous adipose tissue obtained by lipoaspiration or surgical removal; bone marrow obtained by puncture; non-mobilized peripheral blood and peripheral blood after mobilization of bone marrow obtained by venipuncture or apheresis; endometrium, obtained by biopsy of the uterus; menstrual blood; umbilical-cord tissue, Wharton's jelly and umbilical-cord blood obtained after/during birth; amniotic fluid obtained by amniocentesis or during birth by caesarean section; amniotic membrane obtained after birth; tooth pulp, obtained from teeth.

The term "functionalized nanoparticle" refers to a nanoparticle with surface functional groups (e.g. NH2, OH, COOH, SH, etc.) on its surface, enabling the binding to the 3D carrier structure of the membrane and/or the binding of target molecules onto the surface of the functionalized nanoparticle.

The term "biofunctionalized nanoparticle" refers to functionalized nanoparticle having already bound target molecules or parts thereof on its surface that recognize the characteristic antigen on the surface of target stem cell, and can bind to this characteristic antigen.

Functionalized nanoparticles can be inorganic, organic, hybrid, composite, magnetic or combinations thereof; and consist of metals and/or their alloys and/or metal oxides and/or polymers or any combination of the above basic materials featuring surface functional groups (e.g. NH2, OH, COOH, SH, etc.). In one embodiment, functionalized nanoparticles are hybrid inorganic-organic nanoparticles. Preferentially, functionalized nanoparticles are nanoparticles of metal alloys (e.g. NiCu - nickel/copper) enclosed by a layer of silica (SiO₂) having surface functional groups on the surface. Functionalized nanoparticles can also be metal oxides (e.g. Fe2O3 or Fe3O4), equally enclosed by a layer of silica, and having surface functional groups on the surface. Preferentially, the thickness of the silica layer ranges from a couple of nanometers up to several tens of nanometers. In another embodiment, functionalized nanoparticles are nanoparticles based on silica (chemically SiO2) prepared from various siloxane-based precursors (e.g. (3-Aminopropyl) triethoxysilane-APTES, vinyl triethoxy silane-VTES, (3-Mercaptopropyl) triethoxysilane-MPTES...), which can also ensure the presence of the desired functional groups on their surface, namely *in situ,* already during the synthesis of these nanoparticles. In yet another embodiment, functionalized nanoparticles are polysaccharide-based nanoparticles (e.g. chitosan, carboxymethyl cellulose, alginate, etc.). Their basic structure already includes the desired preferential functional groups (e.g. NH2, OH, COOH, etc.) ensuring a similar function as the other exposed functionalized nanoparticle examples.

Another embodiment refers to nanoparticle synthesis based on other synthetic polymers (e.g. dendrimers, derivatives of methacrylates, polyethylenimine, etc.), also including the desired functional groups (e.g. NH2, OH, COOH, SH etc.) in their basic structure, thus also satisfying the initial definition of functionalized nanoparticles. The nanoparticle synthesis can be carried out using the sol-gel method, emulsion techniques, or any other synthesis procedure enabling the preparation of nanoparticles that satisfy the definition of a functionalized nanoparticle consisting of the above mentioned and other basic materials.

The input single-cell suspension is a suspension of individual cells and minor cell clusters, prepared from a biological sample in a physiological buffer, i.e. a buffer enabling the preservation of cells in physiological conditions. Such buffers are for example saline solution, culture medium, 1xPBS (phosphate buffered saline) and other similar solutions. Thereby, the input suspension of cells comprises target stem cells as well as other, non-target cells, i.e. the rest of tissue cells, non-target stem cells, cellular debris, and other components (e.g. blood plasma, intercellular liquid, extracellular matrix) that can be present in a biological sample.

The term "membrane activation" means binding of target molecules, which recognize and bind characteristic antigens on the surface of target stem cells, onto/into the 3D carrier structure of the membrane. Membrane activation can be performed through the inclusion of target molecules using any chemical, physicochemical, or physical method. This includes but is not limited to the inclusion of functionalized nanoparticles into the carrier structure of the membrane and the subsequent binding of target molecules to said nanoparticles, the integration of biofunctionalized nanoparticles into the carrier structure of the membrane or direct binding of target molecules to the carrier structure of the membrane.

### Detailed description of the invention

The invention is described below and presented with embodiments and in the figure.

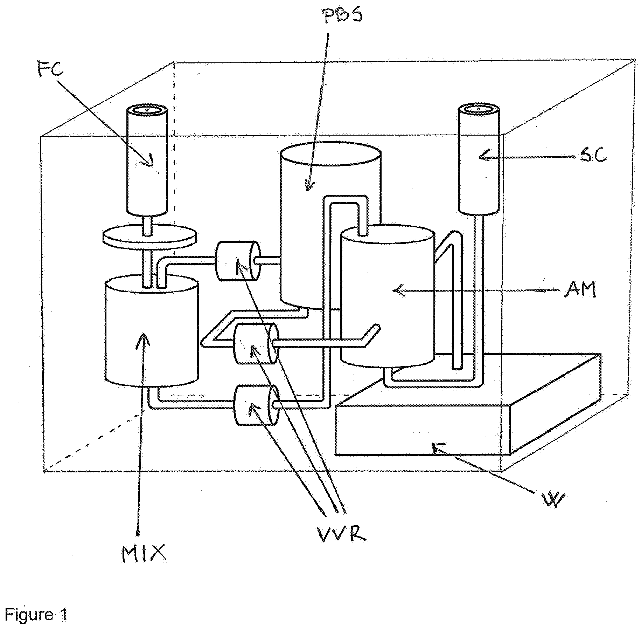

Figure 1 shows an exchangeable cassette, the constituent part of the device for separation of stem cells from biological samples, including the membrane according to the invention.

The membrane according to the invention consists of a 3D carrier structure with included target molecules on the surface and/or in the pores of said carrier structure. Said carrier structure is made of at least one layer of a biocompatible polymer of structured or unstructured geometry, with pores of a diameter in the range from 200 to 500 µm, having on its surface and/or in the pores covalently bound target molecules, which recognize and bind characteristic antigens on the surface of the target stem cells. Thereby, target stem cells are caught onto the membrane whereas non-target cells pass through the membrane or can be rinsed off the membrane surface.

Structured geometry of the individual layer of the carrier structure means that in each individual layer the shape, size and distribution of the pores are uniform throughout the layer. The layer itself can be defined during the production procedure, which can be controlled. Unstructured geometry means that in each individual layer the shape, size and distribution of the pores are coincidental and cannot be influenced during the production. The geometry of the individual layer of the carrier structure depends on the membrane production process used. For example when using 3D printing the geometry of the individual layer of the carrier structure will be structured whereas when using electrospinning the geometry of the individual layer will be for the most part unstructured or coincidental, which is a characteristic of this method.

Suitable biocompatible polymer can be hydrophobic or hydrophilic. Preferentially, it is hydrophobic. Biocompatible polymer should not bind target stem cells; it should be inert towards the target stem cells (meaning that it should not influence their essential characteristics, such as differentiation status and potential, proliferation status and potential, expression of surface antigens); during usage it should not enhance the secretion and/or occurrence of any substances that would have short- or long-term negative impacts on the patient being treated with such cells; it should enable sterilization without changing the polymer characteristics; preferably it should not bind thrombocytes or erythrocytes; it should exhibit specific physicochemical and mechanical characteristics important for producing the membrane, such as adequate viscosity, pKa value, printability, surface tension, etc. Suitable biocompatible polymers include, but are not limited to various woven and nonwoven natural materials, e.g. derivatives of polysaccharides- alginate (ALG), carboxymethyl cellulose (CMC), viscose (VIS), silk, collagen, nanofibrillated cellulose (NFC), etc. and combinations thereof, semisynthetic materials like chitosan (CHI) with derivatives, cellulose and other derivatives as well as combinations thereof; and synthetic materials, e.g. polycaprolactone (PCL), polyethylene terephthalate (PET), polybuthylene terephthalate (PBT), polypropylene (PP), polyhydroxyethylmethacrylate (PHEMA), poly(N-(2-hydroxypropyl)methacrylamide) (PHPMA), polyvinyl alcohol (PVA), polyethylene oxide (PEOX), various dendrimers, e.g. polyamidoamine (PAMAM), polyethylenimine (PEI) etc., and combinations thereof.

Preferentially, biocompatible polymers are selected from PCL, CMC, CHI, ALG, PET, PEOX, and PHEMA/PHPMA. This does not exclude other biocompatible polymers or combinations thereof.

The selection of a biocompatible polymer for the production of the carrier structure itself can ensure the carrier structure to feature functional groups on the surface and/or in the pores (e.g. NH2, OH, COOH, SH, etc.).

When the carrier structure is formed of several layers of structured and/or unstructured geometry, the individual layers can be prepared from the same or different biocompatible polymers, and the geometry of the individual layers, that is, the shape, size and distribution of the pores in the individual layer, can be the same or different.

The geometry of the individual layer is determined in such a way that while target stem cells bind to the surface and/or in the pores of the membrane as many nontarget cells as possible pass through the membrane. Optionally, functionalized nanoparticles can be *"in situ"* covalently (or in another way using any chemical, physicochemical or physical method) bound onto/into the carrier structure composed of the biocompatible polymer, i.e. on the surface or/and in the pores of said carrier structure. Functionalized nanoparticles can be integrated into/onto the carrier structure merely "mechanically", i.e. using no special bonds or interactions with the carrier structure (or are just caught onto/into it). To the functionalized nanoparticles target molecules are covalently bound via the surface functional groups that is via active sites of functionalized nanoparticles.

During the membrane production process, i.e. *"in situ",* biofunctionalized nanoparticles, i.e. functionalized nanoparticles with bound target molecules, can be already integrated in the carrier structure.

According to the invention 3D printing (e.g. extrusion, laser, etc. and combinations thereof) is used for producing membrane. According to the invention, 3D printing technique is used to produce a membrane with structured or "calculatedly" unstructured geometry. For this purpose, it is best to use an extrusion-based 3D printer, which extrudes the polymer or hydrogels by heating/melting and mechanical extrusion. Combinations of all techniques are possible. The 3D printer can simultaneously enable accurate (up to picolitres) pipetting of the chosen active molecules' solution/suspension onto predefined spots on the membrane structure, which represents another method of ensuring desired membrane activation. In addition, the inner structure of the individual extruded filaments can be carried out in a form of tunnels or two/more-layer filaments (i.e. core/shell printing), which enables additional control over chemical, physicochemical and mechanical membrane characteristics as well as control of membrane characteristics in its active or inactive state.

Another biomedical engineering process for the production of 3D membrane however, not covered by the process claims, is electrospinning serving to produce membranes with unstructured or partially structured geometry (resulting macro-materials can always be similar, if desired). Using this method, we can prepare thin layers consisting of multiple sublayers, which can be assembled into a membrane of optional thickness. At the same time, electrospinning can be used to change the surface characteristics of the printed 3D membrane (e.g. enlargement of its specific surface area), the micro- and nano-characteristics of the membrane (e.g. local addition of functional groups featuring electrospun fibers etc.) or even the membrane activation (e.g. if the electrospinning formulation includes biofunctionalized nanoparticles or otherwise integrated target molecules).

Yet another biomedical engineering process for production of 3D membrane however, also not covered by the process claims, is the preparation of woven textiles with structured micro-geometry as well as structured or unstructured micro- and nano-geometry. These are made of infinite fibers or preselected polymers. The latter can be additionally processed using the electrospinning method enabling additional membrane characteristics.

The above processes enable the production of the carrier structure of the membrane from a biocompatible polymer, to which subsequently target molecules are bound. The above processes also enable the production of the carrier structure of the membrane from a biocompatible polymer with already integrated functionalized nanoparticles, to which subsequently target molecules are bound. The above processes enable the production of the carrier structure of the membrane from a biocompatible polymer with already integrated biofunctionalized nanoparticles, as is described below in more details.

Membrane activation or biofunctionalization can be performed directly onto the 3D carrier structure. In this case, the surface of the carrier structure is chemically processed using known methods, e.g. the so called carbodiimide method (CDI) using the reagent 1-Ethyl-3-(3-dimethylaminopropyl) carbodiimide (EDC) which leads to creating amide bonds under "milder conditions", thus obtaining active sites on the surface and in the pores of the carrier structure of the membrane, i.e. covalently bound surface functional groups, to which target molecules (e.g. antibodies, parts thereof, aptamers) covalently bind without or with a minimum influence on their activity. EDC is a water-soluble carbodiimide additionally facilitating covalent binding of active molecules onto the membrane.

The general procedure of such activation includes a reaction in a water medium, into which, besides the reagent EDC, target molecules that we desire to bind (e.g. antibodies), membrane structure and buffer are added. After approx. 30 minutes, a membrane with still active antibodies bound to the carrier structure is obtained, i.e. a membrane able to perform affinity binding of target stem cells which express antigens which recognize antibodies (or target molecules) bound into/onto membrane during the above described process.

Membrane activation can be performed using functionalized nanoparticles, which can be either bound (e.g. covalently) or non-bound (i.e. just "caught") onto/into the carrier structure of the membrane, meaning *"in situ* into a biocompatible polymer, whereby the surface functional groups serve as anchoring points or active sites for the binding of target molecules, which recognize and bind characteristic antigens bound to the surface of the target stem cells, onto the surface of functionalized nanoparticles.

If membrane activation is performed using functionalized nanoparticles, the functionalized nanoparticles (with corresponding functional groups or active sites for binding onto the carrier structure of the membrane and for binding of target molecules which recognize and bind characteristic antigens bound on the surface of the stem cells) are pre-prepared using known methods, e.g. CDI or the amine-reactive cross linker method. The CDI method is primarily used for the activation of carboxyl and phosphate functional groups, the other method mentioned is primarily used for the activation of amine functional groups. The methods can be used simultaneously.

Membrane activation can be performed using functionalized nanoparticles, when the carrier structure of the membrane with integrated functionalized nanoparticles has already been produced by 3D printing technique. In this case, the pre-prepared functionalized nanoparticles, which are prepared in accordance with one of the above described processes, are first integrated into the carrier structure of the membrane according to one of the above described procedures during the production of the carrier structure. Thereby, the functionalized nanoparticles are either *"in situ"* integrated or chemically bound to the carrier structure, on its surface or in the pores.

Functionalized nanoparticles integrated into the carrier structure feature on their surface the above mentioned functional groups (e.g. NH₂, OH, COOH, SH, etc.) which then serve to perform the same chemical binding processes of target molecules (e.g. CDI method) to said functional groups and hence on the carrier structure. Thereupon, membrane activation follows, i.e. the binding of target molecules, which recognize and bind characteristic antigens bound to the surface of stem cells onto the surface of functionalized nanoparticles, by for example following the above membrane activation procedure.

In order to increase the number of active sites on the surface and/or in the pores of the carrier structure with integrated functionalized nanoparticles for the binding of target molecules, the surface of the carrier structure can optionally be processed mechanically (e.g. by grinding, cutting, removing the upper layer of the carrier structure up to the thickness of ~µm) or in another way (e.g. etching). This enables the exposure of a larger quantity of functionalized nanoparticles on the surface or in the pores of the carrier structure, which enhances the efficiency of binding target molecules onto the carrier structure, meaning a larger number of target molecules per membrane surface/volume unit is obtained.

Optionally, the necessary active sites on the surface and/or in the pores of the carrier structure of the membrane for the binding of target molecules - especially in case of woven or electrospun membranes - can be obtained using oxygen plasma ensuring hydrophilicity of the surface and the presence of OH-, COOH- groups on the surface and/or in the pores of the carrier structure; or using nitrogen plasma (or ammonium plasma) ensuring hydrophilicity of the surface and the presence of NH2 functional groups on the surface and/or in the pores of the carrier structure; or using fluoride plasma (or HF) optimizing the hydrophobicity of the carrier structure. Plasma processed surfaces enable the use of the above activation methods as well as further steps of biofunctionalization. For the needs of additional processing of the carrier structure of the membrane, individual plasma processing methods can be repeated or combined.

Membrane activation using functionalized nanoparticles can also be performed prior the membrane preparation, namely in the case, when the membrane is produced using 3D printing, whereby pre-prepared biofunctionalized nanoparticles already featuring target molecules bound to their surface functional groups are added to the melt of the selected biocompatible polymer *»in situ«,* which is followed by membrane production according to one of the above procedures. Thereby, the biofunctionalized nanoparticles with already bound target molecules are integrated into the 3D membrane structure during its production procedure.

The membrane according to invention is produced using the method of 3D printing (bioprinting). The selected biocompatible polymer is melted, and to the melt pre-prepared functionalized nanoparticles with surface functional groups are added *"in situ"*; upon this the 3D membrane structure with structured geometry is produced via 3D printing, whereby functionalized nanoparticles are integrated into the 3D carrier structure of the membrane. Thus produced 3D carrier structure of the membrane is then activated via functionalized nanoparticles with corresponding target molecules, i.e. selected target molecules bind to the functional groups on nanoparticles, that is on the carrier structure of the membrane (thus biofunctionalized nanoparticles in the membrane are obtained). This is how the membrane according to invention is produced.

The membrane according to invention is used in the process for separation/isolation of target stem cells from a biological sample, whereby sterile target stem cells in a physiological buffer with a known cell population size and viability are obtained.

Before entering the membrane, the biological sample is accordingly pre-prepared, i.e. preparing the input single-cell suspension, whereby the adequate size of individual cells in the suspension and the adequate suspension density is ensured.

The preparation of the input single-cell suspension includes known processes for biological sample disintegration and mechanical filtering. The biological sample disintegration processes include, but are not limited to mechanical treatment (e.g., maceration, cutting, scraping, centrifugation), chemical treatment (e.g., treatment with Erythrocyte Lysis Buffer, addition of anticoagulants), enzymatic treatment (e.g. use of collagenase, hyaluronidase, trypsin or combinations thereof), and/or a combination thereof.

Optionally, a step for the erythrocyte removal, for example by using erythrocyte lysis buffer, density-gradient centrifugation, labelled magnetic beads, a method referred to as "buoyant" separation or other known techniques can be added to the preparation of the input single-cell suspension, in particular when the suspension is obtained from the blood and/or from biological samples rich in blood.

The next step in the process is mechanical filtration as a preliminary method for separating particles (cells) from the selected biological sample based not only on the pore size of the filter (particles smaller than the filter pores permeate through the filter), but also on the chemical composition of the filter material. For example, certain substances adhere to the material, from which the selected filter is made of, more than others. Mechanical filtration includes mesh filters of different porosity (e.g., from 10 to 100µm), which are used as single units or in a cascade of successive filters with a decreasing pore size. The chemical composition of the filters may include (but is not limited to) nylon, cellulose acetate, polylactic acid, polyglycolic acid, polyethylene terephthalate, polypropylene, polycaprolactone, provided the mesh filter material does not bind targeted stem cells.

If cascading filters are used, the individual filters in the cascade can be made of different materials. Filters can be comprised of commercially available filters (e.g. Corning^{®} Cell Strainer) and/or in-house developed 3D printed filters or filters produced by means of other techniques and combinations thereof for this purpose. With this pre-preparation of the biological sample, the input single-cell suspension is obtained, whereby the size of individual cells and/or any potential smaller cell clusters in the suspension does not exceed the pore size of the individual membrane in at least two dimensions, and the density of the input suspension of cells is maintained below 2×10⁸ cell/mL, preferably between 1×10⁶ and 1×10⁷ cells/mL.

The appropriate density of the input single-cell suspension is achieved by adding physiological buffer (to achieve dilution) or by increasing the amount of cells in the suspension (through concentration), if necessary. The supply of the input cell suspension to the membrane is controlled automatically. The cell counter (in bioimpedance mode) detects the number of cells approaching the membrane and maintains the input cell suspension density below 2×10⁸ cells/mL by supplying or removing the physiological buffer automatically.

The size of cells and/or any smaller cell clusters in the suspension should not exceed the membrane pore size. The preferred size of individual cells and/or any smaller cell clusters in the suspension in at least two dimensions should not exceed 70µm.

The separation of the target stem cells from the input material, i.e. the biological sample, is carried out on the basis of the free flow of the input single-cell suspension through at least one membrane. During this process, target stem cells are captured on the membrane surface and in the membrane pores due to the specific recognition and binding between the characteristic antigens on the target stem cell surface and target molecules, i.e. antibodies or fragments of antibodies against said characteristic antigens, bound to the membrane. The transition of other non-target cells through the membrane is not hindered (or it is only slightly impeded, for example due to the increased number of bound target stem cells, resulting in reduced effective membrane porosity).

A single membrane can be used to separate target stem cells. However, cells can be also separated using several membranes in a cascade, whereby each subsequent membrane in the cascade has the same or smaller pore dimensions.

The separation process additionally enables multiple filtration of the cell suspension through the membrane / membranes, thereby increasing the efficiency.

Depending on the end-use purpose, target stem cells can be removed from the membrane by one of the below described methods or the membrane together with target stem cells can be used. In the latter case, for example, the membrane can be used as tissue filler to be implanted into the patient experiencing a major trauma. After being placed in a native environment, the membrane-bound stem cells differentiate into desired surrounding tissues and effectively contribute to the regeneration of one or more surrounding tissues. The membrane with bound target stem cells can also be used as a growth substrate to multiply these cells for applying them in a desired way (e.g., in therapy, etc.). Another way of using the membrane with captured target stem cells is to differentiate the cells into an appropriate tissue by means of external stimuli (e.g. by adding selected growth factors to the growth medium or other stimuli). The selection of tissue is, however, limited by the type of captured target stem cells. In this way, a bone segment, for example, can be obtained to be implanted in the patient. These are just a few examples, but there are many more other possibilities of using the membrane with captured target stem cells directly.

The processes for removing target stem cells from the membrane include, but are not limited to: physical/mechanical processes (e.g., pressure variation; increase/ decrease of pressure), physicochemical processes (e.g., ionic strength variation by adding salt, buffers, ultra-pure water rinsing, etc.), biochemical processes (e.g., the use of enzymes, such as peptidases that cleave the bound between membrane and antibodies), chemical processes (e.g., the reduction of disulfide bonds to thiol groups), and affinity processes (e.g. by adding compounds with a greater affinity to the selected active functionalization surface (e.g., antibodies) than cells (which are relatively "large" particles) and other processes and combinations thereof. These procedures for removing target stem cells from the membrane can be used: individually, as a combination of the above-mentioned procedures, as cascade systems of the same or different procedures with any number of further repetitions. Preferential separation procedures minimize the stress and reduce the impact on separated target stem cells to be removed from the membrane, e.g., a process that applies appropriate pressure difference intervals. The selected removal procedures differ according to the properties of the selected membrane, functionalized (or biofunctionalized) nanoparticles, target molecules, and target stem cells. Consequently, in various selected methods/processes of isolating target stem cells from biological samples various removal procedures or combinations thereof can be applied.

The device for separation of target stem cells from a biological sample, i.e. from a single-cell suspension, consists of a housing in which electronic and mechanical components with appropriate regulation are fitted and of an exchangeable cassette. In the electronic part all electronic components necessary for the operation of the device are included, such as an uninterruptible UPS power supply system, sensors for measuring flow rate and temperature in order to ensure and monitor flows and optimal temperature of 37°C suitable for working with biomaterials (said temperature can be adjusted, if necessary), a cell counter, analogue digital converters, electrical converters and the like. In the mechanical part of the device all the mechanical components necessary for the operation of the device are included, such as valve systems, pumps and/or a compressor, opening and closing tracks to insert the exchangeable cassette, fittings to attach the exchangeable cassette to the housing, and a fluid system connection for the exchangeable cassette based on a quick couplings for an easy cassette replacement. The regulation part ensures proper device regulation, thus ensuring optimum device operating conditions, for example adequate regulation of temperature, proper flow regulation to ensure desired concentrations of the input cell suspension.

Optionally, a cleaning cassette can be included in the device to enable self-cleaning, especially when irreplaceable parts are in contact with biological material. The cleaning of the device is applied automatically in accordance with the relevant protocol.

The exchangeable cassette, which is presented in figure 1, comprises a container FC for the input single-cell suspension prepared from a biological sample, a mixing chamber MIX to ensure input suspension density below 2×10⁸ cell/mL by adding, if necessary, physiological buffer from the PBS container, at least one membrane AM of the invention, a waste container W and a collector SC to collect the target stem cell suspension. VVR control valves connected to a single pumping compressor unit in the device ensure proper supply of fluids or suspensions. The exchangeable cassette can be inserted into the device either from the top or from the front.

Raw input material, i.e. the input single-cell suspension can be inserted in the exchangeable cassette located in the container FC as an injection needle or with any other aseptic transportation and storage technique. Adequate density of input single-cell suspension is ensured in the mixing chamber MIX by supplying physiological buffer from the PBS container, if necessary.

The single-cell suspension is then delivered to the membrane AM and the separation of the target stem cells from the input suspension occurs on the basis of the free flow of the input single-cell suspension passing through at least one membrane AM. The target stem cells are bound to the membrane AM, while all non-target cells pass through the membrane AM or are being removed from the membrane surface by rinsing as a residual suspension into the waste container W. Target stem cells are removed from the membrane AM using physiological buffer under pressure supplied from the PBS container through a feedback loop. The resulting suspension of sterile target stem cells in the physiological buffer is collected in the SC collector. This target stem cells suspension can either be immediately applied to the patient or used subsequently for various purposes.

The delivery of the input single-cell suspension to the membrane AM is regulated automatically. The cell counter detects the number of cells reaching the membrane and maintains the density of the input single-cell suspension below 2x10 cells/mL by automatically supplying the physiological buffer from the PBS container.

In the preferred embodiment the cell counter is based on bioimpedance, i.e. two electrodes of any material (silver, etc.) that detect changes in the electrical resistance. The cell counter is installed in two different positions, as follows: at the site before the input single-cell suspension reaches the AM membrane and before the sterile target stem cell suspension in the physiological buffer reaches the collector container SC. An important part of cell counting is their live/dead characterization using a bioimpedance technique to measure the membrane conductivity AM (dead cells have altered conductivity, because of the spilled cytosol) or viability stain, whereby a small sample of cells from the collector container SC is collected online and tested for living cells.

All sensors in the device are connected to the main computer equipped with a touchscreen and user interface (Ul). The device can be connected to the Internet via the main computer and the LAN port. The device can be set up either in a hospital setting, private outpatient clinic, or various research institutes.

The end product of the separation process obtained by the method and the device according to the invention are sterile target stem cells in physiological buffer (as defined in this application) with a known cellular population size (number of isolated cells) and with a known cell viability (live/dead cell ratio).

The end product is intended for use in medical and/or research environments. In particular (but not excluding other possible applications) the following application are possible: direct autologous and allogeneic cell transplantation in patients, experimental or affected animals; cultivation, propagation and cell differentiation in *in vitro* cultures; direct cryopreservation of cells without cultivation for subsequent use; further separation of cell (sub-) populations using other markers.

### Embodiments

### Membrane production

The membrane is made with 3D printing technology. First, a carrier 3D structure of the membrane is made, which consists of ten layers with 100µm pores of polycaprolactone with integrated functionalized nanoparticles of NiCu enclosed by a layer of silica with NH2 functional groups on the surface. Prior to activation (i.e. binding of target molecules), this carrier structure of the membrane undergoes the process of grinding. As a result, more nanoparticles with functional groups are exposed. Separately, a solution of antibodies against CD90 and EDC is prepared, thus activated antibodies are obtained to be bound to the carrier structure of the membrane. The carrier structure is immersed in the activated antibody solution. In about thirty minutes, the antibodies bind to the carrier structure of the membrane, i.e. to the NH2 functional groups. After that, the membrane is rinsed 3-times using deionized water. Now, the membrane is activated and ready to separate target stem cells, in this specific case, stem cells with expressed CD90 antigens.

The procedure for separating target stem cells from a biological sample

### Example 1

### CD34+ hematopoietic stem cell preparation from the bone marrow of a healthy donor for transplantation into a leukemia patient after chemotherapy

A biological sample for the preparation of cells is peripheral blood collected using apheresis following a bone marrow mobilization. The input single-cell suspension is prepared as "buffy coat" - the fraction of a blood sample generated by density gradient centrifugation to remove erythrocytes and blood serum by additional rinsing in 1xPBS.

The final single-cell suspension is prepared in 1l×PBS buffer in an injection. Using an injection needle, the input suspension is applied to a cassette with a 70µm mechanical filter to remove any major cell clusters and a membrane with bound target molecules which recognize and bind the surface antigen CD34 that is expressed on the hematopoietic stem cells.

The regulator of the flow on the membrane ensures optimum dosage of the physiological fluid or buffer to prevent the system from clogging. Once all the non-target cells are collected in the waste container, the efficiency feedback loop ensures that the same solution is re-filtered to capture cells that remained non-bound to the membrane.

The next step includes rinsing of non-target or non-bound cells from the membrane. This is done in a separate feedback loop (not connected to the waste container) using physiological solution or buffer under pressure (e.g. 1 bar or more). Target cells are rinsed and collected in a separate container at the bottom of the device. Rinsed and selected cells are suitable for direct application.

The end product is a sterile CD34+ hematopoietic stem cells suspension suitable for application in patients.

### Example 2

### CD90+ (mesenchymal stem cell) preparation for autologous transplantation

A tumescent lipoaspirate of subcutaneous fat is used as biological sample for the preparation of cells. The input single-cell suspensionis prepared as a stromal vascular fraction (SVF) in accordance with known methods in the following order: rinsing of the lipoaspirate in 1xPBS (erythrocyte and blood serum removal), enzymatic digestion using a collagenase Ia (degradation of intercellular bonds), gradient centrifugation and shaking (final separation of SVF cells and adipocytes), and the removal of adipocytes by pipetting. The final input single-cell suspension is prepared in 1xPBS buffer in an injection.

Using an injection needle, the input suspension is applied to a cassette with a 70 µm mechanical filter to remove any major cell clusters and a membrane with bound target molecules which recognize and bind the surface antigen CD90 that is expressed on the mesenchymal stem cells from subcutaneous fat.

The regulator of the flow on the membrane ensures optimum dosage of physiological fluid or buffer to prevent the system from clogging. Once all the non-target cells are collected in the waste container, the efficiency feedback loop ensures that the same solution is re-filtered to capture cells that remained non-bound to the membrane. The next step includes rinsing of non-target or non-bound cells from the membrane. This is done in a separate feedback loop (not connected to the waste container) using physiological solution or buffer under pressure (e.g. 1 bar or more). Target cells are rinsed and collected in a separate container at the bottom of the device. Rinsed and selected cells are suitable for direct application.

The end product is a sterile CD90+ mesenchymal stem cell suspension suitable for application in patients to treat various medical conditions (orthopedics, cardiology, plastic and reconstructive surgery, stomatology, urology, oncology).

A case of using the single-cell suspension in orthopedics: the obtained CD90+ mesenchymal stem cell suspension is applied directly into the joint space (intra-articular injection) of a joint with surface cartilage damage.

A case of using the single-cell suspension in stomatology: following a tooth extraction, an extensive bone resorption in the jaw occurs. Often this constitutes an obstacle to find an aesthetically appropriate prosthetic solution for the affected jaw and tooth replacement. The optimal solution for the reconstruction of the missing part of the jaw is by using mesenchymal stem cells. In order to ensure a space for bone growth, the existing void must be protected from being overgrown by periosteum. Usually, a titanium mesh is used; however, a 3D printed mesh made of biocompatible material is even better. Stem cells can now be applied to this pre-prepared space. Due to the activity of platelet-derived growth factors, stem cells begin to differentiate into osteoblasts (young bone cells), and, finally, to osteocytes (adult bone cells). Over a few months, the bone defect is bridged with a healthy native tissue.

## Claims

1. A membrane for selective separation of target stem cells from a single-cell suspension containing stem cells, whereby said single-cell suspension is obtained from a biological sample containing stem cells, wherein the membrane consists of a 3D carrier structure made of at least one layer of a biocompatible polymer with pores, wherein said biocompatible polymer is inert towards the target stem cells, meaning that it should not influence essential characteristics of the target stem cells, **characterized in that** the pores have a diameter in the range from 200 to 500 µm for enabling a free flow of the input single-cell suspension through the carrier structure and wherein the carrier structure has on its surface and/or in the pores covalently bound selected target molecules for recognizing characteristic antigens on the surface of the target stem cells to be isolated, and for specific binding to the characteristic antigens on the surface of the target stem cells to be isolated, for selective retrieval of target stem cell subpopulation only, by binding the target stem cells to be isolated to the selected target molecules.

2. The membrane according to claim 1, wherein each individual layer of the carrier structure is either of a structured geometry with a shape, size and distribution of the pores uniform throughout the layer or of an unstructured geometry with a shape, size and distribution of the pores coincidental throughout the layer.

3. The membrane according to claims 1 and 2, wherein when the carrier structure is formed of several layers of structured or unstructured geometry, the individual layers are prepared from the same or different biocompatible polymers and the geometry of individual layers is the same or different.

4. The membrane according to previous claims, wherein the membrane additionally includes functionalized nanoparticles integrated into/onto the membrane structure, i.e. *»in situ«* into a biocompatible polymer from which the carrier structure is made, whereby target molecules are covalently bound onto the functionalized nanoparticles via their surface functional groups, i.e. active sites on the functionalized nanoparticles.

5. The membrane according to previous claims, wherein biocompatible polymers include, but are not limited to various woven and nonwoven natural materials, for example derivatives of polysaccharides - alginate (ALG), carboxymethyl cellulose (CMC), viscose (VIS), silk, collagen, nanofibrillated cellulose (NFC) and others and combinations thereof, to semisynthetic materials like chitosan (CHI) with derivatives, cellulose and other derivatives as well as combinations thereof, and to synthetic materials, for example polycaprolactone (PCL), polyethylene terephthalate (PET), polybuthylene terephthalate (PBT), polypropylene (PP), polyhydroxyethylmethacrylate (PHEMA), poly(N-(2-hydroxypropyl) methacrylamide) (PHPMA), polyvinyl alcohol (PVA), polyethylene oxide (PEOX), to various dendrimers, for example polyamidoamine (PAMAM), polyethylenimine (PEI) and others and combinations thereof, preferably biocompatible polymers are selected from PCL, CMC, HIT, ALG, PET, PEOX and PHEMA/PHPMA.

6. The membrane according to previous claims, wherein said target molecule is an entire antibody or part of the antibody that recognizes the characteristic antigen on the surface of the target stem cell and enables specific binding to the characteristic antigen, preferably, these are antibodies that recognize the following antigens selected from, but not limited to CD90, CD146, CD44, CD73, CD105, CD34, STRO-1, STRO-3.

7. The membrane according to previous claims, wherein functionalized nanoparticles are inorganic, organic, hybrid, composite, magnetic or combinations thereof; and consist of metals or their alloys and/or metal oxides and/or polymers or any combination of the above basic materials and have on their surface functional groups, selected from, but not limited to NH₂, OH, COOH, SH.

8. A process for the production of an activated membrane for separation of target stem cells, wherein said process comprises the following phases:
- pre-preparation of functionalized nanoparticles which have functional groups on their surface for covalently binding the target molecules which recognize and bind characteristic antigens bound to the surface of the stem cells;
- integration of pre-prepared functionalized nanoparticles into/onto the carrier structure during the membrane production process, wherein the carrier structure with the pores having a diameter in the range from 200 to 500 µm is made of a biocompatible polymer inert towards the target stem cells, meaning that it should not influence essential characteristics of the target stem cells , wherein the carrier structure is produced by a 3D printing technique, whereby functionalized nanoparticles are *"in situ"* integrated or chemically bound into the volume, surface and/or the pores of the carrier structure;
- membrane activation, whereby the carrier structure with integrated functionalized nanoparticles is immersed into a solution of target molecules or the active molecules' solution is put by pipetting onto predefined spots on the carrier structure, whereby the target molecules bind to the above functional groups of functionalized nanoparticles.

9. The process according to claim 8, wherein the integration of pre-prepared functionalized nanoparticles into the carrier structure includes melting of the biocompatible polymer, adding previously *"in situ"* prepared functionalized nanoparticles with surface functional groups to the melt, which is followed by the production of the carrier structure, during which the functionalized nanoparticles are integrated into the membrane structure.

10. The process according to claims 8 and 9, wherein the process optionally includes additional processing of the surface of the carrier structure with integrated functionalized nanoparticles to increase the number of active sites on the surface and/or in the pores of the carrier structure, whereby said additional surface processing include mechanical, chemical or plasma processing.

11. The process according to claims 8 to 10, whereby the membrane activation through functionalized nanoparticles can also be performed before the membrane production, whereby to the melt of the selected biocompatible polymer *"in situ"* pre-prepared biofunctionalized nanoparticles with already bound target molecules to their surface functional groups are added, which is followed by the membrane production via 3D printing whereby the biofunctionalized nanoparticles with bound target molecules are integrated in the carrier structure during the production process thereof.

12. A process for the production of an activated membrane for separation of target stem cells, wherein said process includes:
- production of a carrier structure with the pores having a diameter in the range from 200 to 500 µm by a 3D printing technique from a biocompatible polymer(s) inert towards the target stem cells, meaning that it should not influence essential characteristics of the target stem cells, followed by chemical processing of the carrier surface, to obtain covalently bound surface functional groups on the surface and/or the volume and/or in the pores of the carrier structure, or
- production of a carrier structure with the pores having a diameter in the range from 200 to 500 µm by a 3D printing technique from a biocompatible polymer(s) inert towards the target stem cells, meaning that it should not influence essential characteristics of the target stem cells, wherein the selection of the biocompatible polymer itself ensures the carrier structure to feature surface functional groups selected from NH₂, OH, COOH, SH on the surface and/or the volume and/or in the pores of the carrier structure;
- membrane activation, whereby target molecules bind to the above surface functional groups, wherein membrane activation is performed either directly onto said carrier structure during the production of the carrier structure by pipetting of an active molecules' solution onto predefined spots on the carrier structure, or subsequently by immersing said carrier structure into a solution of target molecules.

13. A process for separation of target stem cells from biological samples using the membrane, according to claims 1 to 7, wherein said process comprises:
- preparation of an input single-cell suspension, whereby the input single-cell suspension comprises target stem cells as well as other non-target cells which are other tissue cells, non-target stem cells, cellular debris and other components present in the biological sample, whereby the size of individual cells and/or possible smaller cell clusters in the suspension does not exceed the membrane pore size and the density of the input single-cell suspension is kept below 2×10⁸cells/mL;
- separation of the target stem cells from the input suspension occurs on the basis of the free flow of the input single-cell suspension through at least one membrane, whereby target stem cells are caught onto the membrane surface and in the pores due to the specific recognition and binding between the characteristic antigens on the surface of the target stem cells and target molecules bound to the membrane.

14. The process according to claim 13, wherein the preparation of the input single-cell suspension includes biological sample disintegration processes, which include, but are not limited to mechanical processing, for example e.g. maceration, cutting, scraping, centrifugation, chemical processing, for example e.g. processing with erythrocyte lysis buffer, adding of anticoagulants, enzymatic processing, for example e.g. use of collagenase, hyaluronidase, trypsin or combinations thereof and/or combinations thereof.

15. The process according to claims 13 and 14, wherein the preparation of the input single-cell suspension includes mechanical filtering using mesh filters with porosity between 10 and 100µm, provided the mesh filter material does not bind the target stem cells and whereby mesh filters are used individually or as a cascade of successive filters with a decreasing pore size and whereby individual filters in the cascade are made of different materials.

16. The process according to claims 13 to 15, wherein the preparation of the input single-cell suspension optionally includes a step for the erythrocytes removal before mechanical filtering.

17. The process according to claims 13 to 16, wherein the size of individual cells and/or possible smaller cell clusters in the suspension does not exceed 70µm in at least two dimensions and the density of the input single-cell suspension is between 1×10⁶ and 1×10⁷ cells/mL.

18. The process according to claims 13 to 17, wherein the appropriate density of the input single-cell suspension is achieved by adding physiological buffer to achieve dilution or by increasing their amount in the suspension through concentration, if necessary.

19. The process according to claims 13 to 18, wherein the process includes cell separation using several membranes in a cascade whereby every subsequent membrane arranged in the cascade having the same or smaller pore dimensions.

20. The process according to claims 13 to 18, wherein the process optionally includes removal of target stem cells from the membrane, whereby the processes for removal include, but are not limited to physical or mechanical processes, for example change of pressure; physicochemical processes, for example ionic strength variation by adding salt, buffers, ultra-pure water rinsing; biochemical processes, for example use of enzymes cleaving the bond between the antigen and the membrane; chemical processes, for example reduction of disulfide bonds to thiol groups; affinity processes, for example adding compounds with a greater affinity to the selected active functionalization surface than cells; and combinations thereof, whereby said processes can be used individually or in combination or as cascade systems of same or different processes with any number of repetitions.

21. A device for the separation of target stem cells from the biological sample using the membrane according to claims 1 to 7, wherein said device consists of a housing containing electronic and mechanical components with a corresponding regulation and of an exchangeable cassette, whereby the exchangeable cassette comprises a collection container (FC) for the input single-cell suspension, a mixing chamber (MIX) where by adding physiological buffer from the container (PBS) the density of the input single-cell suspension below 2×10⁸ cells/ml is ensured, if necessary, at least one membrane (AM) for the separation of the target stem cells from the single-cell suspension, a waste container (W) and a collector (SC) for the suspension of the target stem cells.

22. The device according to claim 21, wherein the electronic component includes an UPS power supply, flow and temperature sensors to ensure flow and optimum temperature of 37°C control, a cell counter, analogue to digital converters (ADC) and electrical converters.

23. The device according to claims 21 and 22, wherein the mechanical part of the device includes valve systems, a pump system and/or compressor, a guide system for opening/closing the part where the exchangeable cassette is inserted, clips for fixing the cassette into the device housing as well as a fluid system connection based on speed clips to simplify cassette exchange.

24. The device according to claims 21 to 23, wherein the supply of the input single-cell suspension onto the membrane (AM) is regulated automatically, whereby a cell counter detects the amount of cells and keeps the density of the input suspension below 2×10⁸ cells/mL by automatically adding physiological buffer from the container (PBS).

25. The device according to claims 21 to 24, wherein the cell counter is functioning based on the principle of bioimpedance and consists of two electrodes made of any material detecting the change in electrical resistance, whereby the cell counter is installed in two parts of the device, namely at the site before the input single-cell suspension reaches the membrane (AM) and before the sterile target stem cell suspension in the physiological buffer reaches the container (SC).

26. The device according to claims 21 to 25, wherein the device optionally includes cleaning cassette for self-cleaning, whereby self-cleaning is performed automatically and in accordance with the protocol for automatic self-cleaning.

27. The device according to claims 21 to 26, wherein the sensors are connected with the hub computer with a touchscreen with a correspondent interface and the device is connected to the Internet using the hub computer's LAN port.

## Patentansprüche

1. Membran zur selektiven Trennung von Zielstammzellen aus einer Einzelzell-Suspension enthaltend Stammzellen, wobei die Einzelzell-Suspension aus einer, die Stammzellen enthaltenden biologischen Probe gewonnen wird, wobei die Membran aus einer 3D-Trägerstruktur aus mindestens einer Schicht eines biokompatiblen Polymers mit Poren besteht, wobei das biokompatible Polymer gegenüber den Zielstammzellen inert ist, was bedeutet, dass es wesentliche Eigenschaften der Zielstammzellen nicht beeinflussen sollte, **dadurch gekennzeichnet, dass** die Poren einen Durchmesser im Bereich von 200 bis 500 µm aufweisen, um einen freien Fluss der eingegebenen Einzelzell-Suspension durch die Trägerstruktur zu ermöglichen, und wobei die Trägerstruktur auf ihrer Oberfläche und/oder in den Poren kovalent gebundene ausgewählte Zielmoleküle zur Erkennung charakteristischer Antigene auf der Oberfläche der zu isolierenden Zielstammzellen aufweist, und zur spezifischen Bindung an die charakteristischen Antigene auf der Oberfläche der zu isolierenden Zielstammzellen, durch Bindung der zu isolierenden Zielstammzellen an die ausgewählten Zielmoleküle.

2. Membran nach Anspruch 1, wobei jede einzelne Schicht der Trägerstruktur entweder eine strukturierte Geometrie mit einer Form, Größe und gleichmäßiger Verteilung der Poren in der gesamten Schicht, oder eine unstrukturierte Geometrie mit einer Form, Größe und zufälliger Verteilung der Poren in der gesamten Schicht, aufweist.

3. Membran nach den Ansprüchen 1 und 2, wobei, wenn die Trägerstruktur aus mehreren Schichten einer strukturierten oder unstrukturierten Geometrie gebildet ist, die einzelnen Schichten aus den gleichen oder verschiedenen biokompatiblen Polymeren hergestellt werden und die Geometrie der einzelnen Schichten gleich oder verschieden ist.

4. Membran nach den vorhergehenden Ansprüchen, wobei die Membran zusätzlich funktionalisierte Nanopartikel umfasst, die in der/auf die Membranstruktur integriert sind, d. h. "*in situ"* in ein biokompatibles Polymer, aus dem die Trägerstruktur hergestellt ist, wobei die Zielmoleküle über ihre oberflächenfunktionellen Gruppen, d. h. aktive Stellen auf den funktionalisierten Nanopartikeln, kovalent an die funktionalisierten Nanopartikel gebunden sind.

5. Membran nach den vorhergehenden Ansprüchen, wobei biokompatible Polymere verschiedene gewebte und nicht gewebte natürliche Materialien umfassen, jedoch nicht nur daran beschränkt sind, zum Beispiel Derivate von Polysacchariden-Alginat (ALG), Carboxymethylcellulose (CMC), Viskose (VIS), Seide, Kollagen, nanofibrillierte Cellulose (NFC) und andere sowie Kombinationen davon, halbsynthetische Materialien wie Chitosan (CHI) mit Derivaten, Zellulose und anderen Derivaten sowie Kombinationen davon, und synthetische Materialien, beispielsweise Polycaprolacton (PCL), Polyethylenterephthalat (PET), Polybuthylenterephthalat (PBT), Polypropylen (PP), Polyhydroxyethylmethacrylat (PHEMA), Poly (N-(2-hydroxypropyl) methacrylamid) (PHPMA), Polyvinylalkohol (PVA), Polyethylenoxid (PEOX), verschiedene Dendrimere, beispielsweise Polyamidoamin (PAMAAM), Polyethylenimin (PEI) und andere sowie Kombinationen davon, vorzugsweise werden die biokompatiblen Polymere aus PCL, CMC, HIT, ALG, PET, PEOX und PHEMA/PHPMA ausgewählt.

6. Membran nach den vorhergehenden Ansprüchen, wobei das Zielmolekül ein gesamter Antikörper oder ein Teil des Antikörpers ist, der das charakteristische Antigen auf der Oberfläche der Zielstammzelle erkennt und eine spezifische Bindung an das charakteristische Antigen ermöglicht, vorzugsweise sind dies Antikörper, die die folgenden Antigene erkennen, die ausgewählt, aber nicht beschränkt auf Cd90, Cd146, Cd44, Cd73, Cd105, Cd34, STRO-1, STRO-3 sind.

7. Membran nach den vorhergehenden Ansprüchen, wobei funktionalisierte Nanopartikel anorganisch, organisch, hybrid, komposit, magnetisch oder Kombinationen davon sind; und aus Metallen oder deren Legierungen und/oder Metalloxiden und/oder Polymeren oder einer beliebigen Kombination der obigen Grundmaterialien bestehen und auf ihrer Oberfläche funktionelle Gruppen aufweisen, ausgewählt aus NH₂, OH, COOH, SH, jedoch nicht daran beschränkt.

8. Verfahren zur Herstellung einer aktivierten Membran zur Trennung von Zielstammzellen, wobei das Verfahren die folgenden Phasen umfasst:
- Vor-Vorbereitung funktionalisierter Nanopartikel, die auf ihrer Oberfläche funktionelle Gruppen zur kovalenten Bindung an die Zielmoleküle aufweisen, die charakteristische Antigene, die an der Oberfläche der Stammzellen gebunden sind, erkennen und binden;
- Integrierung von vor-vorbereiteten funktionalisierten Nanopartikeln in/auf die Trägerstruktur während des Membranherstellungsverfahrens, wobei die Trägerstruktur mit den Poren mit einem Durchmesser im Bereich von 200 bis 500 µm aus einem gegenüber den Zielstammzellen inerten biokompatiblen Polymer besteht, was bedeutet, dass es wesentliche Eigenschaften der Zielstammzellen nicht beeinflussen soll, wobei die Trägerstruktur durch eine 3D-Drucktechnik hergestellt wird, wobei funktionalisierte Nanopartikel "*in situ"* in das Volumen, die Oberfläche und/oder die Poren der Trägerstruktur integriert oder chemisch gebunden sind;
- Membran-Aktivierung, wobei die Trägerstruktur mit integrierten funktionalisierten Nanopartikeln in eine Lösung von Zielmolekülen eingetaucht wird oder eine Lösung aus aktiven Molekülen durch Pipettieren auf vordefinierte Stellen auf der Trägerstruktur aufgebracht wird, wobei sich die Zielmoleküle an die obigen funktionellen Gruppen funktionalisierter Nanopartikel binden.

9. Verfahren nach Anspruch 8, wobei die Integrierung von vor-vorbereiteten funktionalisierten Nanopartikeln in die Trägerstruktur das Schmelzen des biokompatiblen Polymers, das Hinzufügen von zuvor "*in situ"* hergestellten funktionalisierten Nanopartikeln mit oberflächenfunktionellen Gruppen zu der Schmelze umfasst, gefolgt von der Herstellung der Trägerstruktur, während der die funktionalisierten Nanopartikel in die Membranstruktur integriert werden.

10. Verfahren nach den Ansprüchen 8 und 9, wobei das Verfahren gegebenenfalls eine zusätzliche Bearbeitung der Oberfläche der Trägerstruktur mit integrierten funktionalisierten Nanopartikeln umfasst, um die Anzahl der aktiven Stellen auf der Oberfläche und/oder in den Poren der Trägerstruktur zu erhöhen, wodurch die zusätzliche Oberflächenverarbeitung eine mechanische, chemische oder Plasmaverarbeitung umfasst.

11. Verfahren nach den Ansprüchen 8 bis 10, wobei die Membranaktivierung durch funktionalisierte Nanopartikel auch vor der Membranherstellung durchgeführt werden kann, wobei zur Schmelze des ausgewählten biokompatiblen Polymers die "*in situ"* vor-vorbereitete biofunktionalisierten Nanopartikel mit bereits gebundenen Zielmolekülen an ihre oberflächenfunktionellen Gruppen zugegeben werden, gefolgt von der Membranherstellung durch 3D-Druck, wobei die biofunktionalisierten Nanopartikel mit gebundenen Zielmolekülen während des Herstellungsverfahrens in die Trägerstruktur integriert werden.

12. Verfahren zur Herstellung einer aktivierten Membran zur Trennung von Zielstammzellen, das Verfahren umfassend:
- Herstellung einer Trägerstruktur mit Poren mit einem Durchmesser im Bereich von 200 bis 500 µm durch eine 3D-Drucktechnik aus einem gegenüber den Zielstammzellen inerten biokompatiblen Polymer(e), was bedeutet, dass es wesentliche Eigenschaften der Zielstammzellen nicht beeinflussen soll, gefolgt von einer chemischen Bearbeitung der Trägeroberfläche, um kovalent gebundene oberflächenfunktionelle Gruppen an der Oberfläche und/oder dem Volumen und/oder in den Poren der Trägerstruktur zu erhalten;
- Herstellung einer Trägerstruktur mit Poren mit einem Durchmesser im Bereich von 200 bis 500 µm durch eine 3D-Drucktechnik aus einem gegenüber den Zielstammzellen inerten biokompatiblen Polymer(e), was bedeutet, dass es wesentliche Eigenschaften der Zielstammzellen nicht beeinflussen soll, wobei die Auswahl des biokompatiblen Polymers selbst gewährleistet, dass die Trägerstruktur oberflächenfunktionelle Gruppen ausgewählt aus NH₂, OH, COOH, SH auf der Oberfläche und/oder dem Volumen und/oder in den Poren der Trägerstruktur aufweist;
- Membranaktivierung, wobei sich Zielmoleküle an die obigen oberflächenfunktionellen Gruppen binden, wobei die Membranaktivierung entweder direkt auf die Trägerstruktur während der Herstellung der Trägerstruktur durch Pipettieren einer Lösung aus aktiven Molekülen auf vordefinierte Stellen auf der Trägerstruktur oder anschließend durch Eintauchen der Trägerstruktur in eine Lösung von Zielmolekülen durchgeführt wird.

13. Verfahren zur Trennung von Zielstammzellen aus biologischen Proben unter Verwendung der Membran nach den Ansprüchen 1 bis 7, das Verfahren umfassend:
- Herstellung einer Eingangs-Einzelzell-Suspension, wobei die Eingangs-Einzelzell-Suspension Zielstammzellen sowie andere Nicht-Zielzellen, die andere Gewebezellen, Nicht-Zielstammzellen, Zelltrümmer und andere in der biologischen Probe vorhandene Komponenten sind, umfasst, wobei die Größe einzelner Zellen und/oder mögliche kleinere Zellcluster in der Suspension die Membranporengröße nicht übersteigt und die Dichte der Eingangs-Einzelzell-Suspension unter 2 × 10⁸ Zellen/ml gehalten wird;
- Trennung der Zielstammzellen aus der Eingangs-Suspension aufgrund des freien Flusses der Eingangs-Einzelzell-Suspension, die durch mindestens eine Membran erfolgt, wobei die Zielstammzellen aufgrund der spezifischen Erkennung und Bindung zwischen den charakteristischen Antigenen auf der Oberfläche der Zielstammzellen und den an die Membran gebundenen Zielmolekülen auf der Membranoberfläche und in den Poren aufgefangen werden.

14. Verfahren nach Anspruch 13, wobei die Vorbereitung der Eingangs-Einzelzell-Suspension Desintegrationsprozesse biologischer Proben umfasst, die eine mechanische Verarbeitung, zum Beispiel Mazeration, Schneiden, Schaben, Zentrifugieren, chemische Verarbeitung, z. B. Bearbeitung mit Erythrozytenlysepuffer, Zugabe von Antikoagulantien, enzymatische Verarbeitung, z. B. Verwendung von Kollagenase, Hyaluronidase, Trypsin oder Kombinationen davon und/oder Kombinationen davon, umfassen, aber nicht darauf beschränkt sind.

15. Verfahren nach den Ansprüchen 13 und 14, wobei die Vorbereitung der Eingangs-Einzelzell-Suspension eine mechanische Filterung unter Verwendung von Maschenfiltern mit einer Porosität zwischen 10 und 100 µm umfasst, vorausgesetzt, dass das Maschenfiltermaterial die Zielstammzellen nicht bindet, und wobei Maschenfilter einzeln oder als Kaskade von aufeinanderfolgenden Filtern mit abnehmender Porengröße verwendet werden und wobei einzelne Filter in der Kaskade aus unterschiedlichen Materialien bestehen.

16. Verfahren nach den Ansprüchen 13 bis 15, wobei die Vorbereitung der Eingangs-Einzelzell-Suspension gegebenenfalls eine Stufe für die Erythrozyten-Entfernung vor der mechanischen Filterung umfasst.

17. Verfahren nach den Ansprüchen 13 bis 16, wobei die Größe einzelner Zellen und/oder möglicher kleinerer Zellcluster in der Suspension 70 µm in mindestens zwei Dimensionen nicht übersteigt und die Dichte der Eingangs-Einzelzell-Suspension zwischen 1 × 10⁶ und 1 × 10⁷ Zellen/ml beträgt.

18. Verfahren nach den Ansprüchen 13 bis 17, wobei die geeignete Dichte der Eingangs-Einzelzell-Suspension bei Bedarf durch Zugabe eines physiologischen Puffers zur Verdünnung oder durch Erhöhung ihrer Menge in der Suspension durch die Konzentration erreicht wird.

19. Verfahren nach den Ansprüchen 13 bis 18, wobei das Verfahren eine Zelltrennung unter Verwendung mehrerer Membranen in einer Kaskade umfasst, wobei jede nachfolgende Membran, die in der Kaskade angeordnet ist, die gleichen oder kleinere Porenabmessungen aufweist.

20. Verfahren nach den Ansprüchen 13 bis 18, wobei das Verfahren gegebenenfalls Entfernung von Zielstammzellen von der Membran umfasst, wobei die Entfernungsverfahren physikalische oder mechanische Verfahren, aber nicht darauf beschränkt sind, beispielsweise Druckänderungen; physikalisch-chemische Verfahren, z. B. lonenstärkevariation durch Zugabe von Salz, Puffern, ultrareines Wasserspülen; biochemische Verfahren, z. B. Verwendung von Enzymen, die die Bindung zwischen dem Antigen und der Membran aufspalten; chemische Verfahren, zum Beispiel Reduktion von Disulfidbindungen zu Thiolgruppen; Affinitätsverfahren, zum Beispiel Hinzufügen von Verbindungen mit einer größeren Affinität zu der ausgewählten aktiven Funktionalisierungsoberfläche als Zellen; und Kombinationen davon, wobei die Verfahren einzeln oder in Kombination oder als Kaskadensysteme gleicher oder verschiedener Verfahren mit einer beliebigen Anzahl von Wiederholungen verwendet werden können.

21. Vorrichtung zur Trennung von Zielstammzellen aus einer biologischen Probe unter Verwendung der Membran nach den Ansprüchen 1 bis 7, wobei die Vorrichtung aus einem Gehäuse besteht, das elektronische und mechanische Komponenten mit einer entsprechenden Regelung und einer austauschbaren Kassette enthält, wobei die austauschbare Kassette einen Sammelbehälter (FC) für die Einzelzell-Suspension umfasst, eine Mischkammer (MIX), wobei durch Zugabe von physiologischem Puffer aus dem Behälter (PBS) die Dichte der Eingangs-Einzelzell-Suspension unter 2 × 10⁸ Zellen/ml gewährleistet wird, falls erforderlich, auch mindestens eine Membran (AM) zur Trennung der Zielstammzellen aus der Einzelzell-Suspension, einen Abfallbehälter (W) und einen Sammler (SC) für die Suspension der Zielstammzellen.

22. Vorrichtung nach Anspruch 21, wobei die elektronische Komponente eine USV-Stromversorgung, Fluss- und Temperatursensoren, um eine Strömung und eine optimale Temperatur von 37°C zu gewährleisten, einen Zellenzähler, Analog-Digital-Wandler (ADC) und elektrische Wandler umfasst.

23. Vorrichtung nach den Ansprüchen 21 und 22, wobei der mechanische Teil der Vorrichtung Ventilsysteme, ein Pumpensystem und/oder einen Kompressor, ein Führungssystem zum Öffnen/Schließen des Teils, an dem die austauschbare Kassette eingesetzt ist, Clips zum Fixieren der Kassette in das Vorrichtungsgehäuse sowie eine Fluidsystemverbindung auf der Basis von Geschwindigkeitsclips zum Vereinfachen des Kassettenaustauschs umfasst.

24. Vorrichtung nach den Ansprüchen 21 bis 23, wobei die Zufuhr der Eingangs-Einzelzell-Suspension auf die Membran (AM) automatisch geregelt wird, wobei ein Zellzähler die Menge an Zellen erfasst und die Dichte der Eingangssuspension unter 2 × 10⁸ Zellen/ml durch automatische Zugabe von physiologischem Puffer aus dem Behälter (PBS) hält.

25. Vorrichtung nach den Ansprüchen 21 bis 24, wobei der Zellzähler auf der Basis des Prinzips der Bioimpedanz funktioniert und aus zwei Elektroden besteht, die aus einem beliebigen Material bestehen, das die Änderung des elektrischen Widerstands erfasst, wobei der Zellzähler in zwei Teilen der Vorrichtung installiert ist, nämlich an der Stelle, bevor die Eingangs-Einzelzell-Suspension die Membran (AM) erreicht und bevor die sterile Zielstammzellsuspension in dem physiologischen Puffer den Behälter (SC) erreicht.

26. Vorrichtung nach den Ansprüchen 21 bis 25, wobei die Vorrichtung gegebenenfalls eine Reinigungskassette zur Selbstreinigung umfasst, wobei eine Selbstreinigung automatisch und nach dem Protokoll zur automatischen Selbstreinigung durchgeführt wird.

27. Vorrichtung nach den Ansprüchen 21 bis 26, wobei die Sensoren mit dem Hub-Computer mit einem Touchscreen mit einer korrespondierenden Schnittstelle verbunden sind und die Vorrichtung mit dem Internet unter Verwendung des LAN-Ports des Hub-Computers verbunden ist.

## Revendications

1. Une membrane pour la séparation sélective de cellules souches cibles à partir d'une suspension unicellulaire contenant des cellules souches, ladite suspension unicellulaire étant obtenue à partir d'un échantillon biologique contenant des cellules souches, la membrane étant constituée d'une structure de support 3D constituée d'au moins une couche d'un polymère biocompatible avec des pores, ledit polymère biocompatible étant inerte vis-à-vis des cellules souches cibles, ce qui signifie qu'il ne doit pas influencer les caractéristiques essentielles des cellules souches cibles, **caractérisée en ce que** les pores ont un diamètre dans la plage de 200 à 500 µm pour permettre un écoulement libre de la suspension unicellulaire d'entrée à travers la structure de support et dans lequel la structure de support a sur sa surface et/ou dans les pores des molécules cibles liées de manière covalente et sélectionnées pour reconnaître des antigènes caractéristiques sur la surface des cellules souches cibles à isoler, et pour une liaison spécifique aux antigènes caractéristiques sur la surface des cellules souches cibles à isoler, pour une récupération sélective de la sous-population de cellules souches cibles uniquement, par la liaison des cellules souches cibles à isoler aux molécules cibles sélectionnées.

2. Membrane selon la revendication 1, dans laquelle chaque couche individuelle de la structure de support est soit d'une géométrie structurée avec une forme, une taille et une distribution des pores uniformes dans toute la couche soit d'une géométrie non structurée avec une forme, une taille et une distribution des pores coïncidents dans toute la couche.

3. Membrane selon les revendications 1 et 2, dans laquelle, lorsque la structure de support est formée de plusieurs couches de géométrie structurée ou non structurée, les couches individuelles sont préparées à partir des mêmes polymères biocompatibles ou de polymères biocompatibles différents et la géométrie des couches individuelles est identique ou différente.

4. Membrane selon les revendications précédentes, dans laquelle la membrane comprend en outre des nanoparticules fonctionnalisées intégrées dans/sur la structure de membrane, c'est-à-dire « in situ », dans un polymère biocompatible à partir duquel la structure de support est réalisée, les molécules cibles étant liées de manière covalente sur les nanoparticules fonctionnalisées par l'intermédiaire de leurs groupes fonctionnels de surface, c'est-à-dire des sites actifs sur les nanoparticules fonctionnalisées.

5. Membrane selon les revendications précédentes, dans laquelle les polymères biocompatibles comprennent, sans y être limités, divers matériaux naturels tissés et non tissés, par exemple des dérivés de polysaccharides-alginate (ALG), de carboxyméthylcellulose (CMC), de viscose (VIS), de soie, de collagène, de cellulose nanofibrillée (NFC) et d'autres et de combinaisons de ceux-ci, des matériaux semi-synthétiques tels que le chitosane (CHI) avec des dérivés, de la cellulose et d'autres dérivés ainsi que des combinaisons de ceux-ci, et des matériaux synthétiques, par exemple la polycaprolactone (PCL), le poly(téréphtalate d'éthylène) (PET), le polybutylène térephtalate (PBT), le polypropylène (PP), le polyhydroxyéthylméthacrylate (PHEMA), le poly (N-(2-hydroxypropyl) méthacrylamide) (PHPMA), l'alcool polyvinylique (PVA), l'oxyde de polyéthylène (PEOX), divers dendrimères, par exemple le polyamidoamine (PAMAM), le polyéthylène-imine (PEI) et autres et des combinaisons de ceux-ci, de préférence des polymères biocompatibles sont choisis parmi PCL, CMC, HIT, ALG, PET, PEOX et PHEMA/PHPMA.

6. Membrane selon les revendications précédentes, dans laquelle ladite molécule cible est un anticorps entier ou une partie de l'anticorps qui reconnaît l'antigène caractéristique sur la surface de la cellule souche cible et permet une liaison spécifique à l'antigène caractéristique, de préférence, ceux-ci sont des anticorps qui reconnaissent les antigènes suivants choisis parmi, mais sans s'y limiter, CD90, CD146, CD44, CD73, CD105, CD34, STRO-1, STRO-3.

7. Membrane selon les revendications précédentes, dans laquelle des nanoparticules fonctionnalisées sont inorganiques, organiques, hybrides, composites, magnétiques ou des combinaisons de celles-ci ; et sont constituées de métaux ou de leurs alliages et/ou d'oxydes métalliques et/ou de polymères ou d'une combinaison quelconque des matériaux de base ci-dessus et ont sur leurs groupes fonctionnels de surface, choisis parmi, mais sans y être limités, NH₂, OH, COOH, SH.

8. Un procédé de production d'une membrane activée pour la séparation de cellules souches cibles, ledit procédé comprenant les phases suivantes :
- la pré-préparation de nanoparticules fonctionnalisées qui ont des groupes fonctionnels sur leur surface pour lier de manière covalente les molécules cibles qui reconnaissent et lient des antigènes caractéristiques liés à la surface des cellules souches ;
- l'intégration de nanoparticules fonctionnalisées pré-préparées dans/sur la structure de support pendant le procédé de production de la membrane, la structure de support avec les pores ayant un diamètre dans la plage de 200 à 500 µm étant constituée d'un polymère biocompatible inerte vis-à-vis des cellules souches cibles, ce qui signifie qu'il ne doit pas influencer les caractéristiques essentielles des cellules souches cibles, la structure de support étant produite par une technique d'impression 3D, les nanoparticules fonctionnalisées étant « in situ » intégrées ou chimiquement liées dans le volume, la surface et/ou les pores de la structure de support ;
- l'activation de la membrane, la structure de support avec des nanoparticules fonctionnalisées intégrées étant immergée dans une solution de molécules cibles ou la solution de molécules actives étant mise par pipetage sur des points prédéfinis sur la structure de support, les molécules cibles se liant aux groupes fonctionnels de nanoparticules fonctionnalisées susmentionnés.

9. Procédé selon la revendication 8, dans lequel l'intégration de nanoparticules fonctionnalisées pré-préparées dans la structure de support comprend la fusion du polymère biocompatible, l'ajout de nanoparticules fonctionnalisées préalablement préparées « in situ » avec des groupes fonctionnels de surface à la masse fusionnée, qui est suivi par la production de la structure de support, pendant laquelle les nanoparticules fonctionnalisées sont intégrées dans la structure de la membrane.

10. Procédé selon les revendications 8 et 9, dans lequel le procédé comprend éventuellement un traitement supplémentaire de la surface de la structure de support avec des nanoparticules fonctionnalisées intégrées pour augmenter le nombre de sites actifs sur la surface et/ou dans les pores de la structure de support, moyennant quoi ledit traitement de surface supplémentaire comprend un traitement mécanique, chimique ou plasma.

11. Procédé selon les revendications 8 à 10, dans lequel l'activation de la membrane à travers des nanoparticules fonctionnalisées peut également être effectuée avant la production de la membrane, moyennant quoi, des nanoparticules biofonctionnalisées pré-préparées « in situ » avec des molécules cibles déjà liées à leurs groupes fonctionnels de surface sont ajoutées à la masse fusionnée du polymère biocompatible sélectionné, ce qui est suivi par la production de la membrane par impression 3D, moyennant quoi les nanoparticules biofonctionnalisées avec des molécules cibles liées sont intégrées dans la structure de support pendant son procédé de production.

12. Procédé de production d'une membrane activée pour la séparation de cellules souches cibles, ledit procédé comprenant :
- la production d'une structure de support dont les pores ont un diamètre dans la plage de 200 à 500 µm par la technique de l'impression 3D à partir d'un/des polymère(s) biocompatible(s) inerte(s) vis-à-vis des cellules souches cibles, ce qui signifie qu'il ne doit pas influencer les caractéristiques essentielles des cellules souches cibles, suivi d'un traitement chimique de la surface de support, pour obtenir des groupes fonctionnels de surface liés de manière covalente sur la surface et/ou le volume et/ou dans les pores de la structure de support, ou
- la production de la structure de support avec les pores ayant un diamètre dans la plage de 200 à 500 µm par la technique de l'impression 3D à partir d'un/des polymère(s) biocompatible(s) inerte(s) vis-à-vis des cellules souches cibles, ce qui signifie qu'il ne doit pas influencer les caractéristiques essentielles des cellules souches cibles, la sélection du polymère biocompatible lui-même garantissant que la structure de support a des groupes fonctionnels de surface choisis parmi NH₂, OH, COOH, SH sur la surface et/ou le volume et/ou dans les pores de la structure de support ;
- l'activation de la membrane, moyennant quoi des molécules cibles se lient aux groupes fonctionnels de surface susmentionnés, l'activation de la membrane étant effectuée soit directement sur ladite structure de support pendant la production de la structure de support par pipetage d'une solution de molécules actives sur des points prédéfinis sur la structure de support, soit par la suite par immersion de ladite structure de support dans une solution de molécules cibles.

13. Procédé pour la séparation de cellules souches cibles à partir d'échantillons biologiques à l'aide de la membrane selon les revendications 1 à 7, ledit procédé comprenant :
- la préparation d'une suspension unicellulaire d'entrée, la suspension unicellulaire d'entrée comprenant des cellules souches cibles ainsi que d'autres cellules non cibles qui sont d'autres cellules de tissu, des cellules souches non cibles, des débris cellulaires et d'autres composants présents dans l'échantillon biologique, la taille des cellules individuelles et/ou d'éventuels groupes de cellules plus petits dans la suspension ne dépassant pas la taille des pores de la membrane et la densité de la suspension unicellulaire d'entrée étant maintenue inférieure à 2 ×10⁸ cellules/mL ;
- la séparation des cellules souches cibles de la suspension d'entrée se produit sur la base de l'écoulement libre de la suspension unicellulaire d'entrée à travers au moins une membrane, moyennant quoi des cellules souches cibles sont piégées sur la surface de membrane et dans les pores en raison de la reconnaissance et de la liaison spécifiques entre les antigènes caractéristiques sur la surface des cellules souches cibles et des molécules cibles liées à la membrane.

14. Procédé selon la revendication 13, dans lequel la préparation de la suspension unicellulaire d'entrée comprend des procédés de désintégration d'échantillon biologique, qui comprennent, mais sans s'y limiter, un traitement mécanique, par exemple une macération, une découpe, un raclage, une centrifugation, un traitement chimique, par exemple un traitement avec un tampon de lyse érythrocytaire, l'ajout d'anticoagulants, un traitement enzymatique, par exemple l'utilisation de collagénase, d'hyaluronidase, de trypsine ou de combinaisons de ceux-ci et/ou les combinaisons de ceux-ci.

15. Procédé selon les revendications 13 et 14, dans lequel la préparation de la suspension unicellulaire d'entrée comprend un filtrage mécanique à l'aide de filtres à mailles avec une porosité comprise entre 10 et 100 µm, à condition que le matériau du filtre à mailles ne se lie pas aux cellules souches cibles, et moyennant quoi des filtres à mailles sont utilisés individuellement ou sous la forme d'une cascade de filtres successifs ayant une taille de pore décroissante et les filtres individuels dans la cascade étant constitués de matériaux différents.

16. Procédé selon les revendications 13 à 15, dans lequel la préparation de la suspension unicellulaire d'entrée comprend éventuellement une étape pour l'élimination des érythrocytes avant la filtration mécanique.

17. Procédé selon les revendications 13 à 16, dans lequel la taille des cellules individuelles et/ou des groupes de cellules plus petits possibles dans la suspension ne dépasse pas 70 µm dans au moins deux dimensions et la densité de la suspension unicellulaire d'entrée est comprise entre 1×10⁶ et 1×10⁷ cellules/mL.

18. Procédé selon les revendications 13 à 17, dans lequel la densité appropriée de la suspension unicellulaire d'entrée est obtenue en ajoutant un tampon physiologique pour obtenir une dilution ou en augmentant leur quantité dans la suspension par concentration, si nécessaire.

19. Procédé selon les revendications 13 à 18, dans lequel le procédé comprend la séparation de cellules à l'aide de plusieurs membranes dans une cascade, chaque membrane suivante étant disposée dans la cascade ayant les mêmes dimensions de pores ou plus petites.

20. Procédé selon les revendications 13 à 18, le procédé comprenant éventuellement l'élimination de cellules souches cibles de la membrane, les procédés d'élimination comprenant, mais sans s'y limiter, des procédés physiques ou mécaniques, par exemple un changement de pression ; des procédés physico-chimiques, par exemple une variation de force ionique par ajout de sel, de tampons, de rinçage à l'eau ultra-pure ; des procédés biochimiques, par exemple l'utilisation d'enzymes clivant la liaison entre l'antigène et la membrane ; des procédés chimiques, par exemple une réduction de liaisons disulfure avec des groupes thiol ; des procédés d'affinité, par exemple l'ajout de composés ayant une affinité supérieure à la surface de fonctionnalisation active sélectionnée par rapport aux cellules; et des combinaisons de ceux-ci, lesdits procédés pouvant être utilisés individuellement ou en combinaison ou en tant que systèmes en cascade de même procédés ou de procédés différents avec un nombre quelconque de répétitions.

21. Un dispositif pour la séparation de cellules souches cibles à partir de l'échantillon biologique à l'aide de la membrane selon les revendications 1 à 7, ledit dispositif étant constitué d'un boîtier contenant des composants électroniques et mécaniques avec une régulation correspondante et d'une cassette échangeable, la cassette échangeable comprenant un récipient de collecte (FC) pour la suspension unicellulaire d'entrée, une chambre de mélange (MIX) où, en ajoutant un tampon physiologique à partir du récipient (PBS), la densité de la suspension unicellulaire d'entrée inférieure à 2×10⁸ cellules/ml est assurée, si nécessaire, au moins une membrane (AM) pour la séparation des cellules souches cibles de la suspension unicellulaire, un récipient à déchets (W) et un collecteur (SC) pour la suspension des cellules souches cibles.

22. Dispositif selon la revendication 21, dans lequel le composant électronique comprend une alimentation électrique UPS, des capteurs d'écoulement et de température pour assurer un écoulement et une température optimale de commande de 37 °C, un compteur de cellules, des convertisseurs analogique-numérique (ADC) et des convertisseurs électriques.

23. Dispositif selon les revendications 21 et 22, dans lequel la partie mécanique du dispositif comprend des systèmes de soupape, un système de pompe et/ou un compresseur, un système de guidage pour ouvrir/fermer la partie où la cassette échangeable est insérée, des attaches pour fixer la cassette dans le boîtier du dispositif ainsi qu'une connexion de système de fluide basée sur des attaches rapides pour simplifier l'échange de cassette.

24. Dispositif selon les revendications 21 à 23, dans lequel l'alimentation de la suspension unicellulaire d'entrée sur la membrane (AM) est régulée automatiquement, moyennant quoi un compteur de cellules détecte la quantité de cellules et maintient la densité de la suspension d'entrée en dessous de 2×10⁸ cellules/mL en ajoutant automatiquement un tampon physiologique à partir du récipient (PBS).

25. Dispositif selon les revendications 21 à 24, dans lequel le compteur de cellules fonctionne sur la base du principe de bio-impédance et est constitué de deux électrodes constituées de tout matériau détectant le changement de résistance électrique, le compteur de cellules étant installé dans deux parties du dispositif, à savoir au niveau du site avant que la suspension unicellulaire d'entrée n'atteigne la membrane (AM) et avant que la suspension de cellules souches cibles stériles dans le tampon physiologique n'atteigne le récipient (SC).

26. Dispositif selon les revendications 21 à 25, le dispositif comprenant éventuellement une cassette de nettoyage pour l'auto-nettoyage, l'auto-nettoyage s'effectuant automatiquement et conformément au protocole d'auto-nettoyage automatique.

27. Dispositif selon les revendications 21 à 26, dans lequel les capteurs sont connectés à l'ordinateur concentrateur par un écran tactile avec une interface correspondante et le dispositif est connecté à Internet à l'aide du port LAN de l'ordinateur concentrateur.
